# EUROPEAN PATENT APPLICATION

(11) **EP 4 285 854 A1**
(43) Date of publication of application: **06.12.2023**
(21) Application number: 22197485.0
(22) Date of filing: 23.09.2022
(51) Int. Cl.: A61B 34/20

(54) **NAVIGATION IN HOLLOW ANATOMICAL STRUCTURES**

(30) Priority: 02.06.2022 US 202263348033 P
(71) Applicant: Koninklijke Philips N.V., 5656 AG Eindhoven (NL)
(72) Inventor: SINHA, Ayushi, Eindhoven (NL); LEE, Brian C., Eindhoven (NL); VARBLE, Nicole, 5656AG Eindhoven (NL); BYDLON, Torre Michelle, 5656AG Eindhoven (NL)
(74) Representative: Philips Intellectual Property & Standards

(57) **Abstract**

The present invention relates to medical navigation. In order to provide facilitated information for navigation in hollow anatomical structures, a device (10) for navigation in hollow anatomical structures is provided that comprises a data input (12), a data processor (14) and an output interface (16). The data input is configured: to provide 3D image data of a hollow structure in a region of interest of a subject, wherein the 3D image data comprises a coordinate space; to provide a current pose of a tool with a tool tip inserted in the hollow structure. The data processor is configured: to transfer the estimated current pose of the tool tip to the coordinate space of the 3D image data based on the registration of the tool tip with the coordinate space of the 3D image data; and to generate, from the 3D image data, a rendered image showing a scene inside the hollow structure relating to the transferred estimated current pose of the tool tip. The output interface is configured to provide the rendered image to a user.

## Description

### FIELD OF THE INVENTION

The present invention relates to medical navigation, and relates in particular to a device for navigation in hollow anatomical structures, to a system for navigation in hollow anatomical structures and to a method for navigation in hollow anatomical structures.

### BACKGROUND OF THE INVENTION

Minimally invasive procedures may be performed under the guidance of navigated imaging devices that can be inserted into the patient body in order to image and/or navigate to a region of interest. Examples include devices with RGB cameras (endoscopes, bronchoscopes, etc.) as well as other imaging modalities such as endobronchial ultrasound (EBUS), intravascular ultrasound (IVUS), optical coherence tomography (OCT), and so forth. Navigated imaging devices are sometimes used in combination with other interventional imaging modalities like X-ray, tomosynthesis or cone-beam computed tomography (CBCT). These modalities capture a large field of view that enables locating the navigated imaging devices within the reference frame of a broader patient anatomy. However, these modalities expose patients and procedural staff to ionizing radiation and, therefore, minimizing their use is critical.

### SUMMARY OF THE INVENTION

There may thus be a need for facilitated information for navigation in hollow anatomical structures.

The object of the present invention is solved by the subject-matter of the independent claims; further embodiments are incorporated in the dependent claims. It should be noted that the following described aspects of the invention apply also for the device for navigation in hollow anatomical structures, for the system for navigation in hollow anatomical structures and for the method for navigation in hollow anatomical structures.

According to the present invention, a device for navigation in hollow anatomical structures is provided. The device comprises a data input, a data processor and an output interface. The data input is configured to provide 3D image data of a hollow structure in a region of interest of a subject, wherein the 3D image data comprises a coordinate space. The data input is also configured to provide a current pose of a tool with a tool tip inserted in the hollow structure. The data processor is configured to transfer the estimated current pose of the tool tip to the coordinate space of the 3D image data based on the registration of the tool tip within the coordinate space of the 3D image data. The data processor is also configured to generate, from the 3D image data, a rendered image showing a scene inside the hollow structure relating to the transferred estimated current pose of the tool tip. The output interface is configured to provide the rendered image to a user.

According to an example, the data input is configured to provide current image data of the region of interest acquired by an interventional imaging device arranged in the hollow structure in a current pose. The current image data comprises image data relating to a tool with a tool tip inserted in the hollow structure. The data processor is configured to register the interventional imaging device in the current pose within the coordinate space of the 3D image data. The data processor is also configured to estimate the current pose of the tool tip visible in the current image data. The data processor is further configured to transfer the estimated current pose of the tool tip from the current image data to the coordinate space of the 3D image data based on the registration of the interventional imaging device within the coordinate space of the 3D image data.

As an effect, a detailed image of the current scene, also referred to as current situation, is rendered within the reference frame of the 3D image data. In an example, the 3D image data is preoperative image data, i.e. image data acquired beforehand. In an example, the scene inside the hollow structure relating to the transferred estimated current pose of the tool tip, is the rendered image representing a field of view from the transferred estimated current pose of the tool tip. In an example, the rendered scene may also include a target lesion along with adaptable transparency for at least part of the structures in the rendered image.

According to an example, for the registration of the interventional imaging device with the coordinate space of the 3D image data, the data processor is configured to estimate a current pose of the interventional imaging device using the current image data of the region of interest acquired by the interventional imaging device.

According to an example, the 3D image data is at least one of the group of: computed tomography (CT) image data, cone-beam computed tomography (CBCT) image data or magnetic resonance (MRI) image data of the subject. In an option, the current image data comprises at least one of the group of: camera image data from an endoscope, colonoscope, or bronchoscope, image data from an ultrasound transducer arrangement or optical coherence tomography image data.

In an example of the device, the data processor is configured to render an image representing a field of view from the tool tip. In an option, the data processor is also configured to render a target lesion in the rendered image and to provide an adaptable transparency for at least a part of the structures in the rendered image.

According to an example, for the registration of the interventional imaging device with the coordinate space of the 3D image data, the data processor is configured to provide initial 3D image data of the interventional imaging device within the region of interest. The data processor is configured to segment the interventional imaging device in the initial 3D image data generating an initial pose. The data processor is also configured to track the interventional imaging device. The data processor is further configured to adapt the initial pose based on the tracking.

According to an example, for the registration of the interventional imaging device with the coordinate space of the 3D image data, the data processor is configured to provide initial 2D image data of the interventional imaging device within the region of interest. The initial 2D image data comprises at least one initial 2D image. The data processor is further configured to segment the interventional imaging device in the at least one initial 2D image. The data processor is also configured to register the at least one initial 2D image with the 3D image data. The data processor is furthermore configured to initialize the segmented interventional imaging device within the coordinate space of the 3D image data providing an initial pose of the interventional imaging device. The data processor is also further configured to track the interventional imaging device and to adapt the initial pose of the interventional imaging device based on the tracking.

According to an example, for the registration of the interventional imaging device with the coordinate space of the 3D image data, the data processor is configured to provide 2D image data from the interventional imaging device within the region of interest. The 2D image data comprises at least two 2D images with different viewing directions. The data processor is further configured to estimate camera pose based on the 2D image data and to triangulate a 3D structure from the at least two images based on the estimated camera pose and the features visible in the at least two images. The data processor is also configured to initially segment structures within the 3D image data. The data processor is further configured to register the triangulated 3D structure with a corresponding structure segmented in the 3D image data and to register and initialize the interventional imaging device in the coordinate space of the 3D image data. The data processor is furthermore configured to track the interventional imaging device and to adapt the initial pose of the interventional imaging device based on the tracking.

According to an example, the data processor is configured to generate a confidence estimate. The confidence estimate relates to at least one of the group of a quality of images used to estimate the pose, of a quality of a pose estimation, and of a quality of registration. The output interface is configured to provide the confidence estimate to the user.

According to the present invention, a system for navigation in hollow anatomical structures is provided. The system comprises an interventional imaging device configured for insertion in hollow structures, a tool with a tool tip configured for insertion in the hollow structure and a device for navigation in hollow anatomical structures according to one of the preceding examples. The system also comprises a display. The interventional imaging device provides the current image data of the region of interest of a subject. The display shows the generated rendered image.

According to the present invention, also a method for navigation in hollow anatomical structures, is provided. The method comprises the following steps:
- Providing 3D image data of a hollow structure in a region of interest of a subject. The 3D image data comprises a coordinate space.
- Providing a current pose of a tool with a tool tip inserted in the hollow structure.
- Transferring the estimated current pose of the tool tip to the coordinate space of the 3D image data based on the registration of the tool tip with the coordinate space of the 3D image data.
- Generating, from the 3D image data, a rendered image showing a scene inside the hollow structure relating to the transferred estimated current pose of the tool tip.
- Providing the rendered image to a user.

According to an example of the method, the following steps are provided:
- Arranging an interventional imaging device in the hollow structure.
- Providing current image data of the region of interest acquired by the interventional imaging device in the current pose; wherein the current image data comprises image data relating to a tool with a tool tip inserted in the hollow structure.
- Registering the interventional imaging device in the current pose with the coordinate space of the 3D image data.
- Estimating the current pose of the tool tip.
- Transferring the estimated current pose of the tool tip from the current image data to the coordinate space of the 3D image data based on the registration of the interventional imaging device with the coordinate space of the 3D image data.

According to an aspect, a system observes tools visible in bronchoscope field of view and outputs the estimated pose of the tool in the bronchoscope coordinate frame. Given a registration between bronchoscope and a preoperative CT, tool-centric views or views representing a field of view from the tool tip can be rendered in the CT coordinate space to improve on current state-of-the-art in visualization. This additional visualization can enable several downstream tasks as listed below.

According to an aspect, the pose of such tools visible in the field of view of the navigated imaging device is estimated and views from the estimated tool tip are rendered in the coordinate frame of a 3D image, e.g. preoperative CT, that is registered to the navigated imaging device. These tool-centric rendered views can in turn enable other downstream tasks as listed below.

According to an aspect, once registered, the navigated imaging device can be visualized in preoperative image space via an overlay or a rendered view in preoperative image space can be generated from the registered imaging device pose. The rendered view allows visualization of the preoperative planned path or other targets in preoperative image space, e.g. target lesion. In an option, the pose of a tool visible in the field of view of the navigated imaging device is estimated. Although these tools do not provide any imaging feedback, since they are visible in the navigated imaging device, the pose of the tool in the coordinate space of the navigated imaging device can be estimated and views can be rendered from the estimated tool tip. These tool-centric rendered views can in turn enable other tasks like determining if the tool can be navigated in a particular direction or if the tool placement is optimal for a subsequent task.

A field of use is any interventional imaging system including but not limited to bronchoscopes, endoscopes, etc. when used with preoperative or intraoperative 3D imaging, e.g. CT, CBCT, etc.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for navigation in hollow anatomical structures.
Fig. 2 shows an example of a system for navigation in hollow anatomical structures.
Fig. 3 shows basic steps of an example of a method for navigation in hollow anatomical structures.
Fig. 4 shows steps of another example of the method of Fig. 3.
Fig. 5 shows a fluoroscopy image with a bronchoscope and a tool inserted through a working channel of the bronchoscope.
Fig. 6 shows an example of a workflow.
Fig. 7 shows another example of a workflow.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

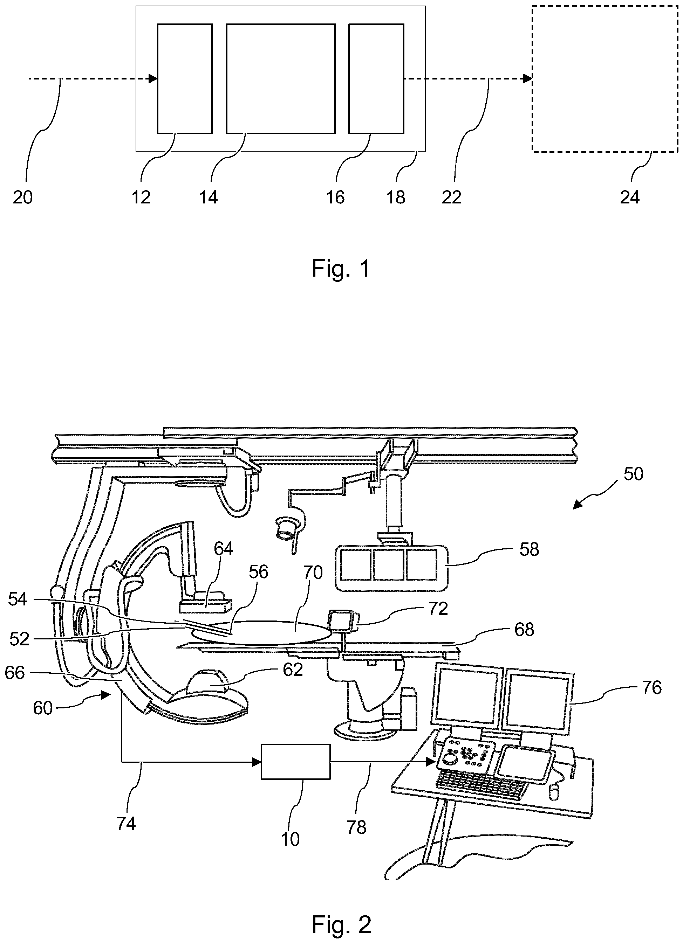

Fig. 1 schematically shows an example of a device 10 for navigation in hollow anatomical structures. The device 10 comprises a data input 12, a data processor 14 and an output interface 16. The data input 12 is configured to provide 3D image data of a hollow structure in a region of interest of a subject. The 3D image data comprises a coordinate space. The data input 12 is also configured to provide a current pose of a tool with a tool tip inserted in the hollow structure. The data processor 14 is configured to transfer the estimated current pose of the tool tip to the coordinate space of the 3D image data based on the registration of the tool tip with the coordinate space of the 3D image data. The data processor 14 is also configured to generate, from the 3D image data, a rendered image showing a scene inside the hollow structure relating to the transferred estimated current pose of the tool tip. The output interface 16 is configured to provide the rendered image to a user.

In an option of the example of Fig. 1, the data input 12 is configured to provide current image data of the region of interest acquired by an interventional imaging device arranged in the hollow structure in a current pose. The current image data comprises image data relating to a tool with a tool tip inserted in the hollow structure. The data processor 14 is configured to register the interventional imaging device in the current pose within the coordinate space of the 3D image data. The data processor 14 is further configured to estimate the current pose of the tool tip visible in the current image data. The data processor 14 is also configured to transfer the estimated current pose of the tool tip from the current image data to the coordinate space of the 3D image data based on the registration of the interventional imaging device within the coordinate space of the 3D image data.

The current pose of the tool with a tool tip is estimated from the current image data of the region of interest acquired by the interventional imaging device arranged in the hollow structure in the current pose.

The term "3D image data" relates to spatial data of the subject which has been acquired by a 3D medical imaging procedure, e.g. 3D ultrasound imaging, computed tomography (CT) imaging or computed tomography angiograph (CTA) imaging or cone-beam computed tomography (CBCT) or 3D rotational angiography (3DRA) or X-ray tomosynthesis imaging or magnetic resonance (MRI) imaging or magnetic resonance angiography (MRA) imaging.

The term "hollow anatomical structure" relates to anatomical structures which are suitable for inserting an interventional device, such as a catheter, endoscope, bronchoscope, or any endovascular or endobronchial device. Examples for a hollow structure are vessels, heart chambers, breathing pathways, i.e. trachea or airways, or the digestive system comprising esophagus, stomach, intestine and colon.

The term "interventional imaging device" relates to a device configured for insertion in a body of a subject and to provide image data of the body when inserted in the body. The interventional imaging device may also be provided for other purposes such as measuring, monitoring or also treatment. In an example, the interventional imaging device is provided as an endoscope or a bronchoscope. In an example, the interventional imaging device is provided as imaging catheter for vascular structures such as optical coherence tomography or intravascular ultrasound.

The term "current image data" relates to image data provided at a current state, e.g. as live images during a medical procedure or intervention. In an example, the image data is provided in an image plane as 2D image data, which can be referred to as current 2D image data. In another example, the image data is provided as 3D image data, which can be referred to as current 3D image data.

The term "pose" relates to location and orientation of e.g. the tool tip or the interventional imaging device, such as the imaging component of the interventional imaging device.

The term "to estimate" relates to the attempt of evaluating and determining the current pose, for example.

The term "to transfer" relates to computing a relation between the two reference frames, also referred to as coordinate spaces, and to determine the respective feature, e.g., the current pose, from the one of the two reference frames, in the other of the two reference frames. The term "reference frame" refers to, for example, in particular to a coordinate space of the current image acquired with the interventional imaging device, or a coordinate space of the 3D image data. The transfer is based on the result of the registration, also referred to as transformation, and relates to defining how the current image data needs to be transformed, i.e. changed in a broad sense, to be aligned with the 3D data set.

The term "coordinate space" relates to the spatial grid or reference system provided for the respective reference frame.

The term "to register" relates to computing the spatial relation of the two different image data sets. The spatial relation comprises information on how to manipulate the respective other data for a spatial matching. The registration comprises rigid registration parts, i.e. a global registration of the 2D image data within the coordinate space of the 3D image data. The registration also comprises non-rigid registration parts, i.e. a morphing or deformable registration process of the 2D image data to the 3D image data. The rigid registration relates to different viewing angles and distances, e.g. caused by movement of a subject support. The non-rigid registration relates to deforming of the subject itself, e.g. caused by breathing or other activity like organ movement comprising in particular the heartbeat.

The term "rendered image" relates to an image that is generated by a graphic process called rendering, which basically means that the 3D surfaces of an object are provided in a 2D image by simulating the interaction of light with objects e.g., using processes like ray tracing. The rendering process may generate images in a somewhat realistic appearance by including photorealistic components like light scatter and surface properties. The rendering process may also generate images showing a pure wireframe model or a wireframe model with hidden lines not shown by excluding the computationally expensive photorealistic components. The rendered image can also be referred to as rendering.

In an example, the image data source is a data storage having stored 3D CT image data of the subject. In an option, the image data source is a CBCT system that is data connected to the device for navigation in hollow anatomical structures during medical procedures.

The term "data input" relates to providing or supplying data for data processing steps. The data input 12 can also be referred to as image data input. The data input 12 can also be referred to as data supply, as image data supply, as image input, as input unit or simply as input. In an example, the image data input 12 is data-connectable to an imaging source arrangement. In an example, the data input 12 is data-connectable to a data storage having stored the image data.

The term "data processor" relates to a processor or part of a processor arrangement that is provided to conduct the computing steps using the data supplied by the data input. The data processor 14 can also be referred to as data processing arrangement, as processor unit or as processor. In an example, the data processor 14 is data-connected to the data input and the output interface.

The term "output interface" relates to an interface for providing the processed or computed data for further purposes. The output interface 16 can also be referred to as output or output unit. In an example, the output interface 16 is data-connectable to a display arrangement or display device. In another example, the output interface 16 is data-connected to a display.

The data input 12, the data processor 14 and the output interface 16 can be provided in a common housing structure, as indicated by a frame 18. They can also be provided in an integrated manner. However, they can also be provided as separate components.

A first arrow 20 indicates data supply to the data input 12. A second arrow 22 indicates data provision from the output interface 16.

In an example, shown as an option in Fig. 1, a display 24 is provided to display the rendered image. In an option, also a current image is shown based on the current image data.

By using the 3D image data for generating the rendered image, the limitation of the imaging devices being able to capture only a small or local field of view within the lumen where they are navigated, is compensated.

In an example, an imaging device is equipped with a working channel through which other devices or tools may be inserted to treat a target.

As an example, if a navigated imaging device is large and cannot be inserted into narrow lumens, the tool can be inserted further while being guided by the interventional imaging device. For example, a bronchoscope may be limited in how far into the airways it can be navigated, for example into the central or intermediate airways, but not into the smaller peripheral airways. Tools and devices that can be inserted into the working channels, on the other hand, are smaller and can be navigated further into smaller lumens. Based on the navigated interventional imaging device, these tools can be navigated toward the target region of interest or even oriented and located relative to the target region of interest.

In an example of the device, the data processor 14 is configured to base the estimation of the current pose of the tool tip on the current image data. In an option, the data processor 14 is configured to generate the rendered image representing a field of view from the tool tip.

In an example, a bronchoscope image processing controller is provided that estimates the current pose of a tool visible in the bronchoscope field of view. This can be done using image processing techniques like segmentation, optical flow, etc. and/or adapting more recent machine/deep learning techniques to the medical domain in order to estimate the 3D pose of articulated tools.

In an example, the estimating of the current pose of the tool tip is based on tracking data from the tool tip. For example, the tracking data is provided as relative tracking data between the tool tip and the interventional imaging device. Tracking data may also be provided using tracking devices like electromagnetic (EM) tracking, shape sensing, etc.

In an example, the rendered image is generated representing a field of view of the interventional imaging device.

In an example, a current distance is calculated between the current pose of the tool tip and a determined target location. The current distance is provided to the user.

In an example, if the location of the target lesion is known, e.g. via a segmentation, then the estimated pose of the tool can be used to compute a distance from lesion metric that can be shown on the user interface. This distance may be computed by estimating the centerline of the airway between the tool location and the lesion location and measuring the length of the centerline (e.g. in mm).

In an example, a device for navigation in hollow anatomical structures is provided, comprising: a data input; a data processor; and an output interface. The data input is configured: to provide 3D image data of a hollow structure in a region of interest of a subject, wherein the 3D image data comprises a coordinate space; to provide current image data of the region of interest acquired by an interventional imaging device arranged in the hollow structure in a current pose, wherein the current image data comprises image data relating to a tool with a tool tip inserted in the hollow structure. The data processor is configured: to register the interventional imaging device in the current pose with the coordinate space of the 3D image data; to estimate a current pose of the tool tip visible in the current image data; to transfer the estimated current pose of the tool tip from the current image data to the coordinate space of the 3D image data based on the registration of the interventional imaging device with the coordinate space of the 3D image data; and to generate, from the 3D image data, a rendered image showing a scene inside the hollow structure relating to the transferred estimated current pose of the tool tip. The output interface is configured to provide the rendered image to a user.

In an example, the scene inside the hollow structure relating to the transferred estimated current pose of the tool tip, is the rendered image representing a field of view from the transferred estimated current pose of the tool tip. Such rendered images may be referred to as tool-centric views. In an example, the rendered scene may also include a target lesion along with adaptable transparency for at least part of the structures in the rendered image.

In an example, for the registration of a device, such as an interventional imaging device or a tool, with the coordinate space of the 3D image data, the data processor 14 is configured to estimate a current pose of the device.

In an example, for the registration of the interventional imaging device with the coordinate space of the 3D image data, the data processor 14 is configured to estimate a current pose of the interventional imaging device using the current image data of the region of interest acquired by the interventional imaging device.

In an example, for the registration of the tool with the coordinate space of the 3D image data, the data processor 14 is configured to estimate a current pose of the tool using tracking devices like EM tracking, shape sensing, etc.

In another example, the 3D image data is CT image data of the subject.

In an option, the current image data comprises at least one of the group of: camera image data from an endoscope or bronchoscope, image data from an ultrasound transducer arrangement or optical coherence tomography image data.

In an example, the interventional imaging device is an endoscope or bronchoscope or other endovascular or endobronchial device.

In an example, in addition or alternatively to interventional imaging devices with RGB cameras, other navigated imaging devices are provided, such as: ultrasound devices like endobronchial ultrasound (EBUS), intravascular ultrasound (IVUS), etc., or optical coherence tomography (OCT) and so forth. As in the examples above, these devices may also be tracked using image processing based methods (if available) or using other tracking devices like EM tracking, shape sensing based tracking, etc.

In an example, for the registration of the interventional imaging device with the coordinate space of the 3D image data, the data processor 14 is configured to provide initial 3D image data of the interventional imaging device within the region of interest. The data processor 14 is also configured to segment the interventional imaging device in the initial 3D image data generating an initial pose. The data processor 14 is further configured to track the interventional imaging device, and to adapt the initial pose based on the tracking.

In an example, for the registration of the interventional imaging device with the coordinate space of the 3D image data, the data processor 14 is configured to provide initial 2D image data of the interventional imaging device within the region of interest. The initial 2D image data comprises at least one initial 2D image. The data processor 14 is configured to segment the interventional imaging device in the at least one initial 2D image. The data processor 14 is configured to register the at least one initial 2D image with the 3D image data. The data processor 14 is configured to initialize the segmented interventional imaging device within the coordinate space of the 3D image data providing an initial pose of the interventional imaging device. The data processor 14 is configured to track the interventional imaging device and to adapt the initial pose of the interventional imaging device based on the tracking.

In an example, the 2D image data of the interventional device may be X-ray images acquired during the procedure. In an example, multiple 2D images from different viewing directions maybe acquired. In an example, multiple 2D images from different viewing directions may be used to perform an image reconstruction to generate an intraoperative 3D tomosynthesis image or 3D cone beam computed tomography image before registering with the initial 3D image data.

In an example, for the registration of the interventional imaging device with the coordinate space of the 3D image data, the data processor 14 is configured to provide 2D image data from the interventional imaging device within the region of interest. The 2D image data comprises at least two 2D images with different viewing directions. The data processor 14 is configured to estimate camera pose based on the 2D image data. The data processor 14 is configured to triangulate a 3D structure from the at least two images based on the estimated camera pose and the features visible in the at least two images. The data processor 14 is configured to initially segment structures within the 3D image data. The data processor 14 is configured to register the triangulated 3D structure with a corresponding structure segmented in the 3D image data. The data processor 14 is configured to register and initialize the interventional imaging device in the coordinate space of the 3D image data. The data processor 14 is configured to track the interventional imaging device. The data processor 14 is configured to adapt the initial pose of the interventional imaging device based on the tracking.

In an option, these examples are applicable also to the tool. In other words, in the example above, the 3D image may contain a tool within the region of interest, which is segmented to provide the initial pose of the tool and the tool is subsequently tracked, e.g. via EM tracking, shape sensing, etc.

In an example, a device is provided, such as an interventional imaging device and/or a tool. The device can be either an interventional imaging device or a tool.

In an example, the data processor 14 is configured to provide the tracking by at least one of the group of: i) relative camera pose estimation based on a sequence of camera images; and ii) at least one of the group of electromagnetic tracking, robotic manipulation data, or shape sensing.

In an example, the data processor 14 is configured to provide the tracking by relative pose estimation based on a sequence of images, such as ultrasound images, OCT images and other imaging devices.

Several of these navigated interventional imaging devices may also be equipped with EM trackers or shape sensed devices, e.g. Fiber Optic RealShape "FORS", that require registration once e.g. at the start of the procedure and can then be tracked in preoperative image space in real-time throughout the procedure.

In an example, current fluoroscopy image data is provided and the data processor 14 is configured to provide an updated estimation of the current pose of the tool tip based on the current fluoroscopy image data.

In an example, for situations when the tool tip is no longer visible in the bronchoscope field of view but visible in subsequent fluoroscopy images, the estimated tool pose from when the tool was visible in bronchoscopy is updated with rough pose estimates from fluoroscopy. Tool pose may be optimized by minimizing errors between fluoroscopy images and synthetic projection images, e.g. digitally reconstructed radiographs (DRRs), generated by updating tool pose in CT space. Tool-centric views can then be generated from the updated tool pose.

In an example, the tools inserted through the working channels of the navigated imaging devices are tracked via EM tracking, shape sensing, gyroscope (orientation), etc. In this case, the bronchoscope image processing controller of the example above is supplemented or replaced by a tracking data processing controller where the tool pose is estimated directly from tracking information. A tool-centric view is then rendered from the computed tool pose as before.

In an example of the device, the data processor 14 is configured: to render a target lesion in the rendered image; and to provide an adaptable transparency for at least a part of the structures in the rendered image.

In an example, the target lesion is also rendered in bronchoscope and/or tool view as long as the lesion is within the field of view, even if it is occluded by other structures. Changing transparency in lesion rendering may indicate the level of occlusion and/or distance from the bronchoscope/tool. That is, when the lesion is far away and occluded by many different anatomical structures, the lesion is rendered with high transparency, whereas when the lesion is close to the bronchoscope/tool, it is rendered with low transparency.

In an example, the data processor 14 is configured to generate a confidence estimate; wherein the confidence estimate relates to at least one of the group of a quality of images used to estimate the pose, of a quality of a pose estimation, and of a quality of registration. The output interface 16 is configured to provide the confidence estimate to the user.

In an example, a confidence estimate is also made along with the pose estimates. Confidence estimates can be made using any method available in the art and can be based on, for instance, the quality of images used to estimate pose (e.g., low confidence if image contains blur, large specular reflections, etc.) and/or the confidence of the pose estimation method itself and/or the quality of the registration with the coordinate space of the 3D image data. For instance, in a neural network based method, confidence in pose estimation may be evaluated using dropout layers in the network which randomly drop the outputs of a specified number of nodes in the network. At inference, pose estimates may be computed multiple times which will result in slightly different pose estimates (due to dropout). The variance in these estimates can be used to estimate the network's confidence (i.e. low variance implies high confidence, high variance implies low confidence). Confidence estimates can also be based on residual errors from registration.

As an effect of an example, navigating tools to target regions of interest without fluoroscopy guidance is enabled. By rendering what could be viewed from the tool tips in CT space, the dependency on fluoroscopy is minimized. Using image based tracking of tools also eliminates reliance on expensive EM tracked or shape sensed tools.

In an aspect, a system is provided that observes tools in the field of view of a bronchoscope (or other navigated interventional imaging device) that is tracked in the coordinate space of a preoperative CT image (or other 3D coordinate space). The pose of the visible tools is estimated in the bronchoscope coordinate frame. The bronchoscope tracking and tool pose estimates are then leveraged to render tool-centric views in the preoperative CT coordinate space in order to enable downstream tasks like determining if the tool can be navigated in the target direction or if tool placement is optimal for biopsy or other tasks, etc.

As an effect, the system can render views from the point of view of tools that do not have cameras at their distal ends.

In an example, an interventional imaging device with an RGB camera is provided that can be navigated into the patient body in order to image and/or access a region of interest, such as: endoscopes, bronchoscopes, and so forth.

Further, a preoperative or intraoperative 3D image of the patient anatomy is provided, such as: CT image, CBCT image, and so forth.

Furthermore, a pre-processing controller is provided that performs an alignment or registration between the interventional imaging device and the 3D image of patient anatomy. This can be done using any of the following methods:
In an option, the bronchoscope visible in intraoperative 3D image acquisitions, e.g. tomosynthesis image, CBCT, etc., is segmented The segmented bronchoscope serves as the initial pose of the bronchoscope, and subsequent bronchoscope tracking is performed using relative camera pose estimates from video-based techniques or tracking devices, such as EM, shape sensing, etc. The relative pose estimates are successively added to the initial bronchoscope pose to track the bronchoscope in the 3D coordinate frame.

In another option, the bronchoscope visible in intraoperative fluoroscopy image acquisitions, e.g. X-ray image or images from multiple views, tomosynthesis, CBCT, etc., is segmented, and the intraoperative image is registered with preoperative CT image. This initializes the segmented bronchoscope in CT space and subsequent bronchoscope tracking can be performed as above.

In another option, camera pose of an interventional imaging device, e.g., bronchoscope, endoscope, etc., is estimated, and the 3D structure visible in the interventional imaging device is triangulated from video frames. A registration between the estimated 3D structure and the corresponding structure segmented in CT (or CBCT, etc.) is provided. Segmentation may only be required for structures that appear at the start of bronchoscope tracking, for instance, the main carina in the airways, in order to register and initialize the bronchoscope in CT space. Subsequent bronchoscope tracking can then be performed as above. This reduces the need for a full airway segmentation in CT.

Finally, a visualization controller takes the estimated tool pose in bronchoscope coordinate space (from above) and the alignment between bronchoscope and CT coordinate spaces (from above) to visualize the tool in CT space. The scene from the point of view of the tool can then be rendered and visualized (as in virtual bronchoscopy but from the point of view of the tool instead of the bronchoscope). The rendering may be performed by segmenting the airways and rendering views within the segmentation using techniques like ray tracing and computation of ray and segmented surface intersection to generate rendered scenes. The rendering may also be performed without segmentations using volume rendering techniques that rely on voxel intensities or grey values to generate rendered scenes. The rendering may be generated with a simulated light source collocated with the virtual camera, i.e. same pose as the virtual camera. The virtual camera may be placed at the same pose as the bronchoscope to generate a virtual bronchoscopy view, or the virtual camera may be placed at the same pose at the tool tip to generate a tool-centric view.

As an option, a postprocessing controller performs downstream tasks based on the resulting tool-centric visualization. Downstream tasks may include (but are not limited to): For example, determining if the tool is positioned in a way that enables navigation in a particular direction is provided. For instance, if the tool must be navigated into an airway branch, then looking down the point of view of the tool can allow physicians to estimate whether pushing the tool further forward will push the tool into an airway branch.

For example, determining if tool placement is optimal for a particular task like acquiring a biopsy is provided. For instance, if a segmented lung lesion is additionally rendered, then visualizing the view from the frame of reference of the tool can allow physicians to determine whether the tool is pointing toward the lesion and whether the current tool positioning will lead to the acquisition of tissue samples from the lesion.

Fig. 2 shows an example of a system 50 for navigation in hollow anatomical structures. The system 50 comprises an interventional imaging device 52 configured for insertion in hollow structures. The system 50 also comprises a tool 54 with a tool tip 56 configured for insertion in the hollow structure. Further, the system 50 comprises an example of the device 10 for navigation in hollow anatomical structures according to one of the preceding and following examples. Further, a display 58 is provided. The interventional imaging device provides the current image data of the region of interest of a subject. The display 58 shows the generated rendered image.

As an option, the interventional imaging device 52 is provided as a bronchoscope. In the example shown, the interventional imaging device 52 comprises a channel for movably receiving the tool 54.

As a further option, also an X-ray imaging system 60 is provided for generating the image data for the 3D image data. The X-ray imaging system 60 comprises an X-ray source 62 and an X-ray detector 64 mounted to ends of a movably supported C-arm 66. In the example shown, the X-ray imaging system 60 is supported by a ceiling mounted rail structure. In another example, the X-ray imaging system 60 is provided as a mobile X-ray imaging system. A subject support 68 is provided, on which a subject 70 is arranged. A control panel 72 is provided next to the subject support 68.

The device 10 for navigation in hollow anatomical structures is data connected to the X-ray imaging system 60, as indicated with a first data connection line 74.

A console 76, e.g. for controlling the various functions of the various devices is provided comprising displays, keyboard, mouse, tablet and control panels. The device 10 for navigation in hollow anatomical structures is data connected to the console 76, as indicated with a second data connection line 78.

In an example, the interventional imaging device 52 is provided as at least one of the group of: an endoscope providing camera image data, a bronchoscope providing camera image data, a colonoscope providing camera image data, an ultrasound transducer arrangement providing ultrasound image data, and an optical coherence tomography arrangement providing optical coherence tomography image data.

In an option, the tool 54 is provided as at least one of the group of: a biopsy device, a guidewire, a needle and a brush.

For example, the endoscope, the bronchoscope, or the colonoscope comprise a RGB camera operating with visible light.

For example, the ultrasound transducer generates ultrasound waves and receives wave signals reflected by surrounding anatomical structures.

For example, optical coherence tomography uses low-coherence light waves to capture micrometer-resolution images. This results in 2D and 3D images from within optical scattering media such as tissue. Optical coherence tomography is based on low-coherence interferometry. An example of application is near-infrared light.

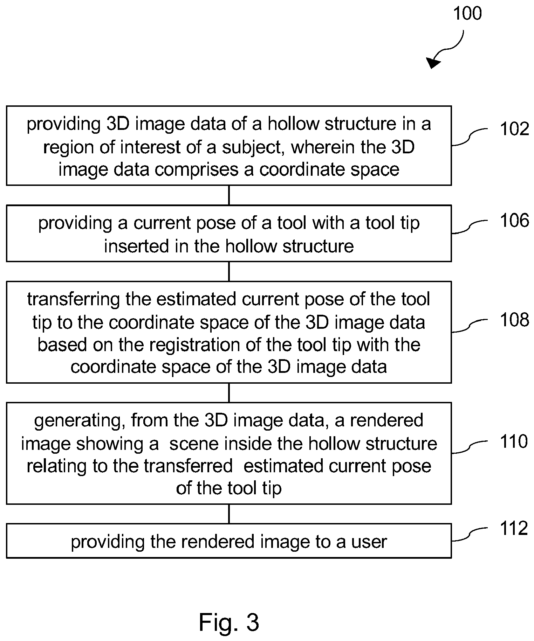

Fig. 3 shows basic steps of an example of a method 100 for navigation in hollow anatomical structures. The method 100 comprises the following steps: In a first step 102, 3D image data of a hollow structure in a region of interest of a subject is provided. The 3D image data comprises a coordinate space. In a second step 106, a current pose of a tool with a tool tip inserted in the hollow structure is provided. In a third step 108, the estimated current pose of the tool tip is transferred to the coordinate space of the 3D image data based on the registration of the tool tip with the coordinate space of the 3D image data. In a fourth step 110, from the 3D image data, a rendered image is generated showing a scene inside the hollow structure relating to the transferred estimated current pose of the tool tip. In a fifth step 112, the rendered image is provided to a user.

In an option, a further step is provided after the first step 102 and before the second step 106, in which further step, an interventional imaging device is arranged in the hollow structure.

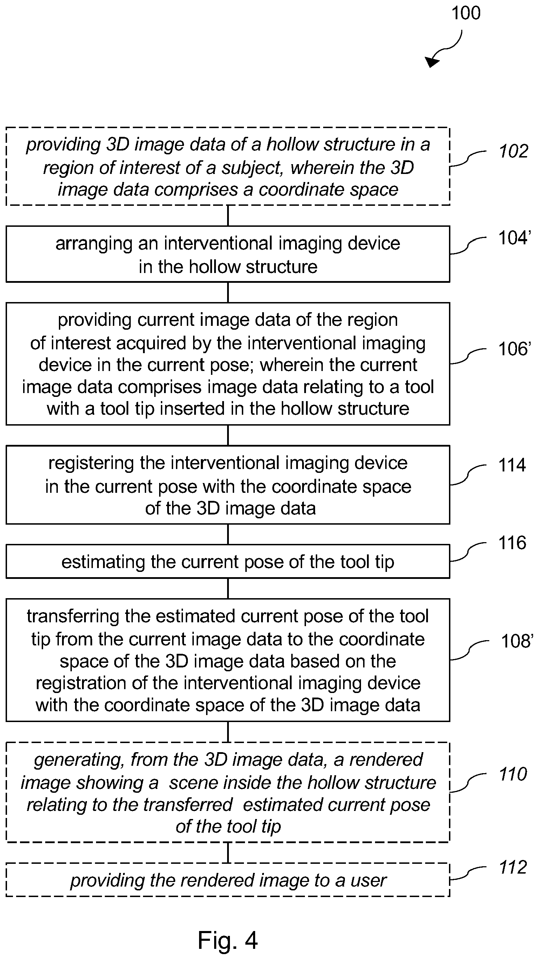

Fig. 4 shows steps of another example of the method of Fig. 3. The following steps are provided comprising the steps:
- Arranging 104' an interventional imaging device in the hollow structure. In an example, this is provided following the first step of the example in Fig. 3.
- Providing 106' current image data of the region of interest acquired by the interventional imaging device in the current pose. The current image data comprises image data relating to a tool with a tool tip inserted in the hollow structure. In an example, this is provided before the registering.
- Registering 114 the interventional imaging device in the current pose with the coordinate space of the 3D image data.
- Estimating 116 the current pose of the tool tip. In an example, this is provided following the providing of the current image data.
- Transferring 108' the estimated current pose of the tool tip from the current image data to the coordinate space of the 3D image data based on the registration of the interventional imaging device with the coordinate space of the 3D image data. In an example, this is provided following the estimating.

Further, the step of generating the rendered image of the example in Fig. 3 and the step of providing the rendered image to the user of the example in Fig. 3 are provided.

In an example, a method for navigation in hollow anatomical structures is provided that comprises the following steps: providing 3D image data of a hollow structure in a region of interest of a subject, wherein the 3D image data comprises a coordinate space; arranging an interventional imaging device in the hollow structure; providing current image data of the region of interest acquired by the interventional imaging device in the current pose, wherein the current image data comprises image data relating to a tool with a tool tip inserted in the hollow structure; registering the interventional imaging device in a current pose within the coordinate space of the 3D image data; estimating a current pose of the tool tip; transferring the estimated current pose of the tool tip from the current image data to the coordinate space of the 3D image data based on the registration of the interventional imaging device with the coordinate space of the 3D image data; generating, from the 3D image data, a rendered image showing a scene inside the hollow structure relating to the transferred estimated current pose of the tool tip; and providing the rendered image to a user.

In an example of the method, the estimating of the current pose of the tool tip is based on the current image data. In an option, the rendered image is generated representing a field of view from the tool tip.

In an example of the method, for the registration of the interventional imaging device with the coordinate space of the 3D image data, the step of estimating a current pose of the interventional imaging device using the current image data of the region of interest acquired by the interventional imaging device is provided.

In an example of the method, the 3D image data is at least one of the group of: CT image data or CBCT image data of the subject. In an option, the current image data comprises at least one of the group of: camera image data from an endoscope or bronchoscope or colonoscope, image data from an ultrasound transducer arrangement or optical coherence tomography image data.

In an example of the method, for the registering of the interventional imaging device with the coordinate space of the 3D image data, it is provided the steps of: providing initial 3D image data of the interventional imaging device within the region of interest; segmenting the interventional imaging device in the initial 3D image data generating an initial pose; tracking the interventional imaging device; and adapting the initial pose based on the tracking.

In an example of the method, for the registering of the interventional imaging device with the coordinate space of the 3D image data, it is provided the steps of: providing initial 2D image data of the interventional imaging device within the region of interest; wherein the initial 2D image data comprises at least two initial 2D images with different viewing directions; segmenting the interventional imaging device in the at least two initial 2D images; registering the at least two initial 2D images with the 3D image data; initializing the segmented interventional imaging device within the coordinate space of the 3D image data for providing an initial pose of the interventional imaging device; tracking the interventional imaging device; and adapting the initial pose of the interventional imaging device based on the tracking.

In an example of the method, for the registering of the interventional imaging device with the coordinate space of the 3D image data, it is provided the steps of: providing 2D image data of the interventional imaging device within the region of interest, wherein the 2D image data comprises at least two 2D images with different viewing directions; estimating camera pose based on the 2D image data; triangulating a 3D structure from the at least two images based on the estimated camera pose and the segmented structures; initial segmenting of structures within the 3D image data; registering the triangulated 3D structure with a corresponding structure segmented in the 3D image data; registering and initializing the interventional imaging device in the coordinate space of the 3D image data; tracking the interventional imaging device; and adapting the initial pose of the interventional imaging device based on the tracking.

In an example of the method, the tracking is provided by at least one of the group of: i) relative camera pose estimation based on a sequence of camera images; and ii) at least one of the group of electromagnetic tracking, robotic manipulation data, and shape sensing.

In an example of the method, current fluoroscopy image data is provided. An updated estimate of the current pose of the tool tip based on the current fluoroscopy image data is provided.

In an example of the method, a target lesion is rendered in the rendered image. An adaptable transparency is provided for at least a part of the structures in the rendered image.

In an example of the method, a confidence estimate is generated and provided to the user. The confidence estimate relates to at least one of the group of a quality of images used to estimate the pose, of a pose estimation method, and a quality of registration.

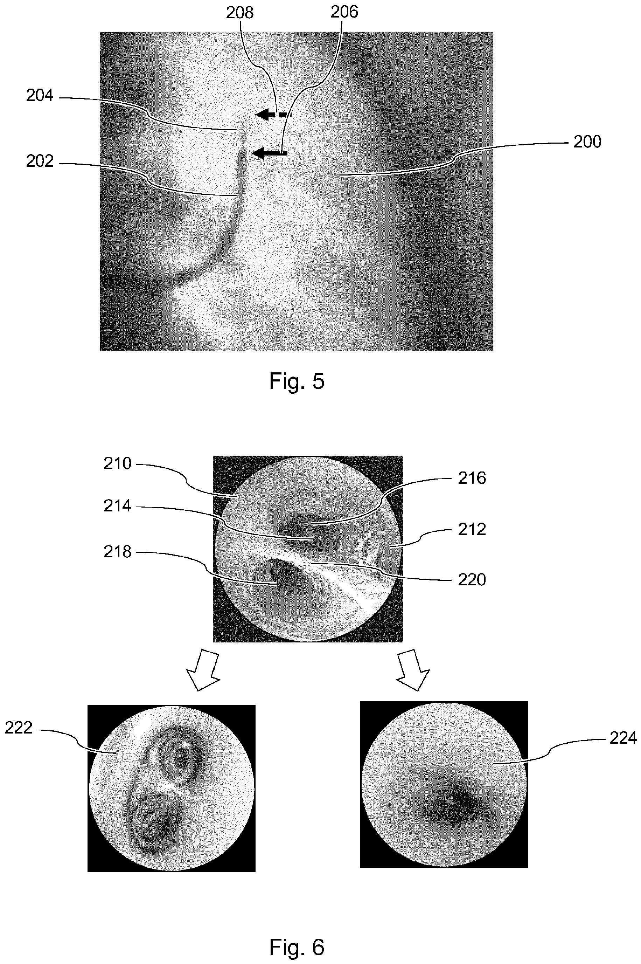

Fig. 5 shows a fluoroscopy image 200 with a bronchoscope 202 and a tool 204 inserted through a working channel of the bronchoscope 202. A first arrow 206 indicates a distal end of the bronchoscope 202. An imaging device is mounted at the distal end. A second arrow 208 indicates a distal end of the tool 204.

Fig. 6 shows an example of a workflow. A bronchoscope provides a bronchoscopic image 210 of a hollow anatomical structure. The bronchoscopic image 210 shows a tool 212 inserted through a working channel of the bronchoscope. The tool 212 with a tool tip 214 reaches further into the hollow anatomical structure. The hollow anatomical structure is shown having a first path 216 and a second path 218 branching of and separated by a wall structure 220.

As a result of the above-described steps, a first rendered image 222 is provided showing the location as seen from the bronchoscope. Further, a second rendered image 224 is provided showing the location as seen from the tool tip 214.

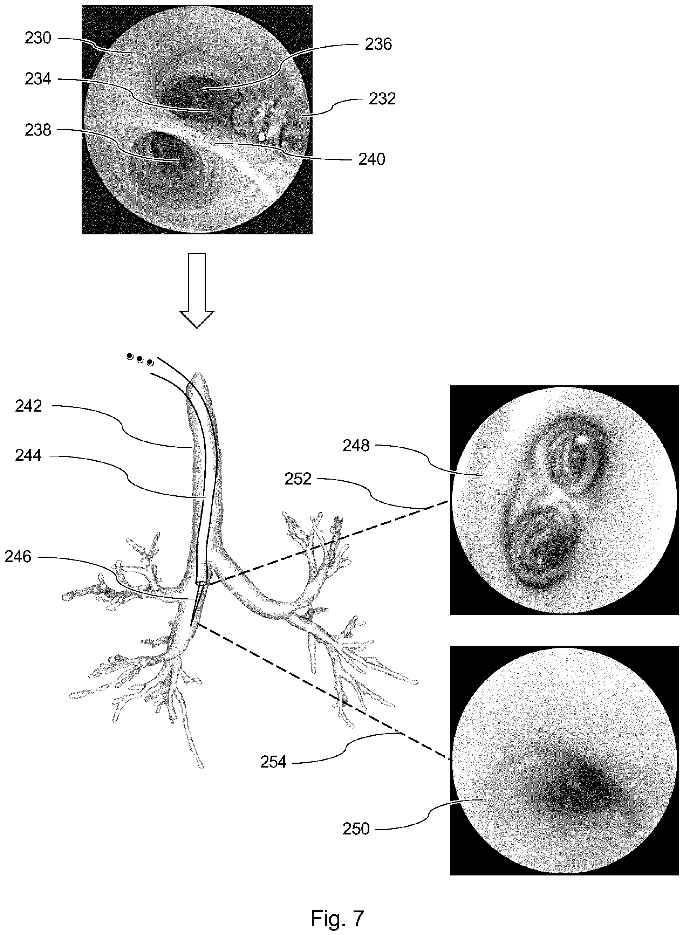

Fig. 7 shows another example of a workflow. Similar to Fig. 6, a bronchoscope provides a bronchoscopic image 230 of a hollow anatomical structure, as indicated in an upper part of the figure. The bronchoscopic image 230 shows a tool 232 inserted through a working channel of the bronchoscope. The tool 232 with a tool tip 234 reaches further into the hollow anatomical structure. The hollow anatomical structure is shown having a first path 236 and a second path 238 branching of and separated by a wall structure 240.

A center part of the figure indicates a representation of an anatomical structure 242, i.e. a segmented airway, in which a bronchoscope 244 is partly inserted. A tool 246 with its distal end is reaching out of a working channel at a distal end of the bronchoscope 244. The bronchoscope 244 is thus having a view with the tool 246 in its field of view. The bronchoscope 244 and the tool 246 are tracked in CT space as explained above. From the two rendered images in the right part of the figure, a top-right rendering 248 shows a bronchoscope view while a bottom-right rendering 250 shows a tool-centric rendering. A first broken line 252 points to a location within the anatomical structure, for which the rendered image 248 is provided. A second broken line 254 points to a location within the anatomical structure, for which the rendered image 250 is provided.

In an example, a computer program comprises instructions which, when the program is executed by a computer, cause the computer to carry out the method of the examples above.

In an example, a computer program or program element for controlling an apparatus according to one of the examples above is provided, which program or program element, when being executed by a processing unit, is adapted to perform the method steps of one of the method examples above.

In an example, a computer readable medium is provided having stored the computer program of the preceding examples.

The term "subject" may also be referred to as individual. The "subject" may further also be referred to as patient, although it is noted that this term does not indicate whether any illness or disease is actually present with the subject.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for navigation in hollow anatomical structures, comprising:
- a data input (12);
- a data processor (14); and
- an output interface (16);
wherein the data input is configured: to provide 3D image data of a hollow structure in a region of interest of a subject, wherein the 3D image data comprises a coordinate space; to provide a current pose of a tool with a tool tip inserted in the hollow structure;
wherein the data processor is configured: to transfer the estimated current pose of the tool tip to the coordinate space of the 3D image data based on the registration of the tool tip with the coordinate space of the 3D image data; and to generate, from the 3D image data, a rendered image showing a scene inside the hollow structure relating to the transferred estimated current pose of the tool tip; and
wherein the output interface is configured to provide the rendered image to a user.

2. Device according to claim 1, wherein:
wherein the data input is configured: to provide current image data of the region of interest acquired by an interventional imaging device arranged in the hollow structure in a current pose, wherein the current image data comprises image data relating to a tool with a tool tip inserted in the hollow structure; and
wherein the data processor is configured: to register the interventional imaging device in the current pose with the coordinate space of the 3D image data; to estimate the current pose of the tool tip visible in the current image data; to transfer the estimated current pose of the tool tip from the current image data to the coordinate space of the 3D image data based on the registration of the interventional imaging device with the coordinate space of the 3D image data.

3. Device according to claim 1 or 2, wherein, for the registration of the interventional imaging device with the coordinate space of the 3D image data, the data processor is configured to estimate a current pose of the interventional imaging device using the current image data of the region of interest acquired by the interventional imaging device.

4. Device according to one of the preceding claims, wherein the 3D image data is at least one of the group of: CT image data, CBCT image data or MRI image data of the subject; and
wherein the current image data comprises at least one of the group of: camera image data from an endoscope or bronchoscope, image data from an ultrasound transducer arrangement or optical coherence tomography image data.

5. Device according to one of the preceding claims, wherein, for the registration of the interventional imaging device with the coordinate space of the 3D image data, the data processor is configured: to provide initial 3D image data of the interventional imaging device within the region of interest; to segment the interventional imaging device in the initial 3D image data generating an initial pose; to track the interventional imaging device; and to adapt the initial pose based on the tracking.

6. Device according to one of the preceding claims, wherein, for the registration of the interventional imaging device with the coordinate space of the 3D image data, the data processor is configured: to provide initial 2D image data of the interventional imaging device within the region of interest; wherein the initial 2D image data comprises at least one initial 2D image; to segment the interventional imaging device in the at least one initial 2D image; to register the at least one initial 2D image with the 3D image data; to initialize the segmented interventional imaging device within the coordinate space of the 3D image data providing an initial pose of the interventional imaging device; to track the interventional imaging device; and to adapt the initial pose of the interventional imaging device based on the tracking.

7. Device according to one of the preceding claims, wherein, for the registration of the interventional imaging device with the coordinate space of the 3D image data, the data processor is configured: to provide 2D image data from the interventional imaging device within the region of interest, wherein the 2D image data comprises at least two 2D images with different viewing directions; to estimate camera pose based on the 2D image data; to triangulate a 3D structure from the at least two images based on the estimated camera pose and the features visible in the at least two images; to initially segment structures within the 3D image data; to register the triangulated 3D structure with a corresponding structure segmented in the 3D image data; to register and initialize the interventional imaging device in the coordinate space of the 3D image data; to track the interventional imaging device; and to adapt the initial pose of the interventional imaging device based on the tracking.

8. Device according to one of the claims 5 to 7, wherein the data processor is configured to provide the tracking by at least one of the group of:
i) relative pose estimation based on a sequence of images; and
ii) at least one of the group of electromagnetic tracking, robotic manipulation data, and shape sensing.

9. Device according to one of the preceding claims, wherein current fluoroscopy image data is provided; and
wherein the data processor is configured to provide an updated estimation of the current pose of the tool tip based on the current fluoroscopy image data.

10. Device according to one of the preceding claims, wherein the data processor is configured: to generate a confidence estimate; wherein the confidence estimate relates to at least one of the group of a quality of images used to estimate the pose, of a quality of a pose estimation, and of a quality of registration; and
wherein the output interface is configured to provide the confidence estimate to the user.

11. A system (50) for navigation in hollow anatomical structures, comprising:
- an interventional imaging device (52) configured for insertion in hollow structures;
- a tool (54) with a tool tip (56) configured for insertion in the hollow structure; and
- a device (10) for navigation in hollow anatomical structures according to one of the preceding claims; and
- a display (58);
wherein the interventional imaging device provides the current image data of the region of interest of a subject; and
wherein the display shows the generated rendered image.

12. A method (100) for navigation in hollow anatomical structures, comprising the following steps:
- providing (102) 3D image data of a hollow structure in a region of interest of a subject, wherein the 3D image data comprises a coordinate space;
- providing (106) a current pose of a tool with a tool tip inserted in the hollow structure;
- transferring (108) the estimated current pose of the tool tip to the coordinate space of the 3D image data based on the registration of the tool tip with the coordinate space of the 3D image data;
- generating (110), from the 3D image data, a rendered image showing a scene inside the hollow structure relating to the transferred estimated current pose of the tool tip; and
- providing (112) the rendered image to a user.

13. Method according to claim 12, comprising the steps:
- arranging (104') an interventional imaging device in the hollow structure;
- providing (106') current image data of the region of interest acquired by the interventional imaging device in the current pose; wherein the current image data comprises image data relating to a tool with a tool tip inserted in the hollow structure;
- registering (114) the interventional imaging device in the current pose within the coordinate space of the 3D image data;
- estimating (116) the current pose of the tool tip; and
- transferring (108') the estimated current pose of the tool tip from the current image data to the coordinate space of the 3D image data based on the registration of the interventional imaging device within the coordinate space of the 3D image data.

14. Computer program comprising instructions which, when the program is executed by a computer, cause the computer to carry out the method of claim 12 or 13.

15. Computer readable medium having stored the computer program of claim 14.
